# EUROPEAN PATENT SPECIFICATION

(11) **EP 3 991 713 B1**
(45) Date of publication and mention of the grant of the patent: **13.09.2023**
(21) Application number: 21198996.7
(22) Date of filing: 24.09.2021
(51) Int. Cl.: A61K 8/02, A61K 8/25, A61K 8/27, A61Q 15/00

(54) **AUMENTED EFFICACY TALC**
TALK MIT ERHÖHTER WIRKSAMKEIT
TALC À EFFICACITÉ AUGMENTÉ

(30) Priority: 29.10.2020 IT 202000025774
(43) Date of publication of application: 04.05.2022
(73) Proprietor: Prolabin & Tefarm S.r.l., 06134 Perugia (IT)
(72) Inventor: SPOGLI, ROBERTO, 06126 PERUGIA (IT); CARDELLINI, FABIO, 06057 MONTE CASTELLO DI VIBIO (PG) (IT); SISANI, MICHELE, 06134 PERUGIA (IT)
(74) Representative: Cutropia, Gianluigi

(56) References cited:
- WO-A1-2006/083387
- FR-A- 1 486 857
- GB-A- 2 113 706
- US-A- 6 132 744

## Description

The present invention relates to a method for the production of a surface modified talc and its application as a cosmetic raw material for deodorant and antiperspirant formulations. Said surface modified talc is characterised by a higher efficacy than an unmodified talc. This higher efficacy is due to a higher ability to absorb the sweat components that generate bad smell, to a higher dispersion speed in hydrophilic phases and to the reaching of better sensorial properties to cosmetic formulations.

Nowadays antiperspirant and/or deodorant products have become widely employed, and are mainly used for the armpit area and feet care. These products are based on solid substances able to absorb sebum and decrease the sweat production, among these solids talc is one of the most employed for its low cost and its easy availability.

Talc is a material known for its sebum absorbing properties. It is used in deodorant products with anti-smell properties. Talc is an inorganic lamellar solid with chemical formula Mg₃Si₄O₁₀(OH)₂.

Talc is able to absorb the sebum produced by the human body, but has a low affinity for the aqueous part of sweat. This problem limits talc specific efficiency as a cosmetic ingredient in deodorant products and therefore it has to be used in association to other ingredients such as antiperspirant products that are able to reduce or block the sweating. The classical antiperspirant materials formulated with talc are made of inorganic aluminium salts, organic aluminium salts and aluminium based minerals.

In the last years, aluminium salts and minerals are under observation because their perspiring properties are linked to the effect of sweat pore occlusion. This occlusion stops the natural skin sweating and often leads to a comedogenic effect.

During the formulation process talc, due to its low water affinity, can't be directly used in water based formulations, but it has to be dispersed in specific dispersing fatty phases, such as natural oils, esters or waxes. Therefore, the production process for these formulations has to be conducted at high temperatures, needed to fuse the fatty phase, resulting in energy consumption.

Considering the previously exposed considerations, it would be useful and desirable to have on the cosmetic market a specific talc that determines a substantial improvement towards the aforementioned problems, thus characterized by a high absorbing ability for the aqueous part of sweat, easily dispersible in the aqueous phase of cosmetic formulations and able to selectively capture fatty acids and the other acidic molecules that are in sweat and are responsible for the bad smell. This talc specialty, characterized by high efficiency, would allow to avoid or reduce the content of classical antiperspirants used in cosmetic formulations, including aluminium hydrochloride, leading to obtain deodorant cosmetic formulations at the same time efficient and safer for human health. Moreover, this specialty of talc, due to its higher efficiency, allows a reduction of the quantity of talc used in the cosmetic formulation of deodorants, giving space to introduce other ingredients in formula.

US006132744A describes a talc surface modification method by deposition of metallic silicates, silica, metal hydroxyl carbonates, metals carbonates and metals aluminates. The deposition method of these substances on the talc surface has been developed aiming to improve the material wettability and ability to retain the perfume present in the formulation. However, this method doesn't require the prior activation of the talc surface with a treatment able to make the raw material more similar to the materials that have to be deposited. The superficial deposition of these substances moreover doesn't produce a talc with a specific absorbent ability towards the molecules responsible for the bad smells, but is based on a non-specific absorption.

US009968529B2 describes a solid blend of clays, talc and alum. This blend is used to increase the alum suspensibility in deodorant product and consequently reduce the concentration of other ingredients needed to realize the formulation. The method doesn't take into account the possibility to obtain, with a specific process of surface activation, a synergic effect between talc and another material, aiming to delete or reduce the concentration of aluminium salts in the cosmetic formulations or to increase the talc absorption efficacy.

There are numerous other talc surface treatment processes described in the known art, however these are different, distinct, or another thing compared to the object of the present invention for at least one, or more than one or every one of the following reasons:
- do not perform the activation of the talc particles by chemical erosion treatment;
- do not directly contemplate the use of clays or lamellar solids with ionic exchange capacity to allow the selective capture of the acids secreted from sweat and the anionic molecules of the sweat;
- do not contemplate the lamellar solids deposition on the talc particles surface;
- no treatment has as target to obtain a synergic effect between talc and zinc hydroxide chloride that obtains simultaneously
   i) the ability to selectively capture the acids secreted from sweat and the anionic molecules of sweat,
   ii) the increase of the absorbing capacity of the aqueous phase of sweat,
   iii) the increase of the absorbing capacity of the oily phase of sweat.

The talc surface treatments to which the state of art refers to are those conducted with the following materials: organosilanes, organophosphates, organosolphurs or their blends; hydrocarbyl phosphonic acids such as n-octadecylphosphonic acid; methicones such as dimethicone, triethoxysilane, lauroyl lysine, phosphates of fluorinated alcohols, salts of myristic acid, polyhydroxystearic acid, perfluoro octyl, microscrystalline cellulose, polyacrylic acid derivatives, poly - oxyalkylenes such as POA, PAG and PAO, polymethylene oxide, polypropylene oxide, polybutilene oxide and their combinations, propylene and ethylene glycols or their mixtures, polyethers modified with polysiloxanes, variously modified polysiloxanes, ethoxylated alkylamines and alkylamines, polyalkylene glycols such as PAG, PEG and their blends with amines and siloxanes.

Purpose of this invention is to delete the inconveniences of the known technique, giving a surface modified talc with higher absorbing efficiency towards the aqueous and fatty components of the sweat and an irreversible of the acidic organic substances of sweat responsible for bad smell.

Another purpose of this invention is to provide such a surface modified talc which is versatile and suitable to be used in cosmetic product for topical use.

Yet another purpose of this invention is to provide such a surface modified talc which is reliable and not harmful for topical use.

These purposes are achieved in accordance with the invention with the characteristics listed in the attached independent claim 1.

Advantageous embodiments appear from the dependent claims.

The talc with a surface modified according to the invention is obtained through two sequential procedures:
a) activation of the talc surface through chemical erosion;
b) deposition on the talc particles surface of specific quantities of lamellar particles able to exchange ions, made of zinc hydroxy chloride.

The result of this procedure is a high efficiency talc that can be used as a cosmetic raw material with a high absorbing capacity towards the bad smell generating components of sweat.

This surface modified talc has been surprisingly obtained through a two steps chemical process using as main row materials an uncoated talc form usually used in the cosmetic deodorant sector and a cationic lamellar clay known in its natural form as simonkolleite or with its chemical name of zinc hydroxy chloride.

The two sequential steps that surprisingly led to obtain the surface modified talc are:
a) activation of the talc surface obtained through chemical erosion using acids, able to modify the surface morphology;
b) deposition on the talc particles surface of specific quantities of lamellar cationic particles made of zinc hydroxy chloride.

The surface modified talc has the following chemical-physical characteristics:
- average particle size (D₅₀) between 1-30 µm,
- spheroidal particles morphology detectable by scanning electronic microscope SEM,
- surface are between the values of 1-30 m²/g,
- zinc hydroxide chloride lamellar particles that partially coat the surface of the talc particles and that are visible through SEM-EDX morphological analysis,
- XRD crystallographic signals in which there are and are clearly distinguishable the signal of the peak at 2Θ = 9.4° ± 0.4 interlayer distance = 9.4 ± 0.4 Å typical for talc and the signal of the peak at 2 θ = 11.2° ± 0.4, interlayer distance = 7.9 ± 0.4 Å typical for zinc hydroxide chloride.

The talc superficially modified according to the invention can be used in the in cosmetic applications in the field of deodorants and antiperspirants, with special reference to its greater efficiency in absorbing the oily and / or aqueous phase of sweat compared to an untreated talc.

Moreover, the talc superficially modified according to the invention can selectively capture acidic or anionic sweat molecules via an ionic exchange mechanism performed by the anionic lamellar clay that is deposited on the talc particles surface.

The talc superficially modified according to the invention has a higher dispersion speed in hydrophilic phases compared to a surface unmodified talc.

Further characteristics of the invention will be clearer after the detailed description that follows, referred to exemplary and non-limiting embodiments, illustrated in the attached drawings, in which:
Fig. 1 contains an SEM scanning electron microscope image of the talc superficially modified according to the method of the invention, the EDX spectrographic images of zinc (in green) and magnesium (in red) and superimposition of the SEM and EDX images in which they are highlighted the zinc hydroxy chloride particles deposited on the surface of the talc particle. All images refer to the same particle.
Fig. 2 is an image illustrating the monophasic crystal structure of zinc hydroxy chloride characterized by an XRPD crystallographic pattern in which a first reflection (d₀₀₃) at 2Θ = 11.1° ± 0.4 with an interlayer distance at 7.9 ± 0.4 Å is highlighted.
Fig. 3 is a comparative histogram of the absorption efficiency between the talc according to the invention, zinc hydroxy chloride, the reference talc and the reference talc after acid or basic treatment.
Fig. 4 is an XRPD crystallographic pattern of talc according to the invention, which highlights a reflection at 2Θ = 9.4° ± 0.4 that can be attributed to talc and a reflection at 2Θ = 11.1° ± 0.4 that can be attributed to zinc hydroxy chloride.

In the context of the present invention, the following definitions, descriptions and measurement techniques will be used with the meanings illustrated below:
Lamellar solid is an inorganic compound not having organic carbon, that, by its chemical structure, forms a solid that spatially arrange itself as a succession of lamellae, that is, of planar macrocrystals that have a much smaller size (thickness) than the other two (see chapter 1 of Volume VII of Comprehensive Supramolecular Chemistry, Pergamon Press, Oxford, 1996). If the lamellae have an electric charge (positive or negative), in the galleries, that is to say between the lamellae, ions of opposite charge get set, in order to preserve the electric neutrality of the solid. These ions can be substituted by other ions through ion exchange reaction by concentration effect or as a consequence of a higher affinity towards the lamellar matrix of some ions compared to others.

Ionic exchange capacity (IEC): is a dimension defined by the quantity of ions that can be exchange in a certain material. For lamellar solids with positive charges located on the lamella we speak specifically of anion exchange capacity (AEC) as the exchange reactions take place by the anions present in the interlayer region and on the surface of the solid. The AEC is expressed as milliequivalents per gram (meq/g), or as milliequivalents per 100 g (meq/100 g) of material (see Surface and Interface Chemistry of Clay Minerals, Volume 9, 2018, Editors: Robert Schoonheydt Cliff Johnston Faïza Bergaya in Developments in Clay Science).

Ion exchange reaction: consists in the isomorphic substitution of one ion with another on the surface, in the interlayer region or in the interstices of a crystal. This substitution doesn't affect the crystalline structure of the solid (see Whitworth T.M. (1998) Clay minerals: Ion exchange. In: Geochemistry. Encyclopaedia of Earth Science. Springer, Dordrecht).

Intercalation: reversible inclusion or insertion of molecules or ions in the interlayer region of a material with lamellar structure.

Intercalation compound: is the product obtained after intercalation or ionic exchange reactions of a lamellar solid. Ionic exchange reactions, or intercalation reactions, consist in the replacement of ions that balance the charge of the lamellae by other ions that come in contact with the lamellar solid. These reactions are kinetically and thermodynamically driven by factors such as: ions relative concentration, temperature and specific affinity of the ion for a lamellar structure. Each ion has a specific affinity for a certain lamellar solid and this characteristic makes some lamellar solids extremely selective towards some ion categories.

Zinc hydroxy chloride: it is a lamellar solid, belonging to the species of the lamellar anionic clays with chemical formula Zn₅(OH)₈Cl₂ . xH₂O (0.5<x<4). It can also be found indicated by the names of tetrabasic zinc chloride, basic zinc chloride or zinc hydroxychloride. It is a white / colourless solid and insoluble in water, it occurs naturally in the mineral called simonkolleite. The powder X-ray diffraction spectrum of the material has a first characteristic reflection (d003) at 2Θ = 11.2° corresponding to the interlayer distance = 7.9 ± 0.4 Å. Zinc hydroxy chloride is able to capture large quantities of anionic substances through ion exchange reactions including those present in sweat such as nicotinic acid, formic acid, propionic acid, butyric or isobutyric acid, valeric and isovaleric acid, caproic acid, caprylic acid, E-3-methyl-2-hexenoic acid and 3-hydroxy-3-methyl-hexanoic acid and amino acids including cysteine. The AEC of zinc hydroxy chloride is between 3-4 meq / g depending on the chemical composition of the solid. The carboxylic acids mentioned above have high chemical affinity and selectivity for zinc hydroxy chloride and are able to give intercalation reactions by exchanging the chloride anion present in the interlayer region of the clay. Taking into account as an example the caprylic acid that has a molecular mass of 144,22 g/mol, 1 gram of zinc hydroxy chloride is able to capture through intercalation reactions a quantity in mass of this acid between 0.43-0.58 g, corresponding to the quantity of fatty acids contained in 10 litres of sweat (artificial reconstituted sweat).

As a consequence of the capture or intercalation of the acidic components of sweat in the lamellar solid following ion exchange reactions, there is a significant decrease in the substances that are the main cause of bad odour. However, zinc hydroxyl chloride has a low absorbency of sweat and sebum, compared to that of talc.

Artificial sebum: it is a fluid that simulates the fatty phase of sweat and it is made of linseed oil. Linseed oil is composed similarly to human sebum and it is also used as reference oil in EN ISO 787-5:1995 for the determination of the capacity of powders to absorb oil.

ISO artificial sweat: it is a saline solution that simulates the aqueous phase of sweat. It is obtained according to the indications of ISO 105-B07: 2009 and has the following composition expressed as weight percentage: sodium chloride 0.5%; histidine 0.05%; sodium dihydrogen phosphate 0.22%; water 99.23%. ISO artificial sweat simulates the sweat aqueous component and is used to determine the non-specific ability of a material to absorb the aqueous phase of sweat.

Reconstituted artificial sweat: it is a saline solution that simulates the aqueous phase of sweat. It is obtained according to the indications described in the academic article "Harvey, Christopher J., Ryan F. LeBouf, and Aleksandr B. Stefaniak. "Formulation and stability of a novel artificial human sweat under conditions of storage and use." Toxicology in Vitro 24.6 (2010): 1790-1796 ". It has the following composition expressed as weight percentage: sodium chloride 0.18%; lactic acid 0.13%; nicotinic acid 5.0%; urea 0.06%; arginine 0.14%; water 94.49%. Reconstituted artificial sweat is used to evaluate the ability of a material to selectively absorb some classes of organic molecules contained in sweat.

The composition of artificial sweat has been simplified compared to that described in literature, by inserting in the saline solution only the component with the highest concentration for each class of substances. The classes of substances are the following: 1) organic acids; 2) vitamins; 3) substances containing nitrogen; 4) amino acids. Therefore, lactic acid is representing organic acids, nicotinic acid has been chosen for vitamins, urea for substances containing nitrogen and histidine for amino acids. These four substances were used in the reconstituted artificial sweat.

In the tests carried out with reconstituted artificial sweat, the ability of the material to selectively absorb the nicotinic acid has been taken as a reference. Nicotinic acid was evaluated as the most representative compound among organic acid molecules in sweat and mainly responsible for the development of bad odor, due bacterial and enzymatic degradation. Nicotinic acid is also to be considered representative for the following acid molecules present in sweat: formic acid, propionic acid, butyric or isobutyric acid, valeric and isovaleric acid, caproic acid, acid caprylic, palmitic acid, stearic acid, oleic acid, linoleic acid, α-linolenic acid, arachic acid, gadoleic acid, E-3-methyl-2-hexanoic acid and 3-hydroxy-3-methyl-hexanoic acid and amino acids including cysteine.

Cosmetic composition indicates a composition that is intended to be applied to the human body to beautify, promote attractiveness or alter the appearance without affecting the structure or functions of the body. Specifically, according to the present invention, the cosmetic composition has the purpose of reducing the odor produced by body perspiration. This odor reduction can be obtained with specific components contained in the cosmetic formulation including: components that absorb the oily phase and / or the aqueous phase of sweat, components that occlude the hair pores preventing perspiration such as aluminum hydrochloride, components that act at an enzymatic level such as triethyl citrate or antibacterial materials, which aim is to reduce the effects due to the microbial digestion of the sweat components by the bacteria present on the skin.

A cosmetic composition can be realized in different ways, it could be an anhydrous powder (e.g., pressed or loosened), a liquid, a gel, an A / O emulsion, an O / A emulsion, a detergent, a shampoo, a solution micellar, dispersion or anhydrous stick.

The term "cosmetic composition", as defined in the Cosmetic Regulation (EC Reg. No. 1223/2009 and subsequent amendments. Mm. li.), means "any substance or mixture intended to be applied on the external surfaces of the human body, hair system and hair, nails, lips, external genital organs) or on the teeth and mucous membranes of the mouth, for the sole or primary purpose of cleaning, perfuming, modifying their appearance, protecting them, keeping them in good condition or correcting body odors".

Specifically, according to the present invention, the cosmetic composition has the purpose of reducing the odor produced by the natural perspiration of the body. This odor reduction can be obtained with specific components contained in the cosmetic formulation including: absorbents of the oily phase and / or the aqueous phase of sweat, antiperspirants that clog the hair pores preventing perspiration such as aluminum hydrochloride, substances that act at the same level enzymatic such as triethyl citrate or antibacterials, which aim is to reduce the effect produced by the microbial digestion of the sweat components by the skin bacteria.

The deodorant cosmetic compositions can be prepared in different forms, including dry powder, liquid, gel, emulsion, emulgel, cream and aerosol.

Artificial sebum absorption test: the test is conducted according to EN ISO 787-5: 1995 on a powder material to determine the mass amount of linseed oil that the powder is able to absorb. The test is conducted using a watch glass where 5 g of powdered product are placed. A 25 ml calibrated burette is then filled with linseed oil and brought to volume. Few drops of linseed oil are dripped onto the powder, then powder and oil drops are mixed with a spatula until reaching a homogeneous mixture. This operation is repeated until a spreadable paste is obtained. The quantity of oil absorbed per gram of powder is calculated by the volume of oil added and the density value.

ISO artificial sweat absorption test: the test is conducted on a powder material to determine the quantity by mass of ISO artificial sweat that this powder is able to absorb. The execution of the test requires to use three centrifuge tubes containing 2 g of powder product. For each tube 15 ml of ISO artificial sweat are added. The suspensions are left under magnetic stirring overnight. The day after the tubes are centrifuged at 15000 rpm for 15 minutes. The supernatant is removed and the remaining solid is weighted. The test is repeated in triplicate on the same sample.

Reconstituted artificial sweat absorption test: the test is conducted on a powdered material to determine the mass amount of nicotinic acid that this powder is able to capture. The execution of the test requires to use three centrifuge tubes containing 2 g of powder product. For each tube, 15 ml of reconstituted artificial sweat is added. The suspensions are left under magnetic stirring overnight. The next day the tubes are centrifuged at 15000 rpm for 15 minutes. The remaining solid is weighed. The supernatant is instead transferred to a volumetric flask and suitably diluted. The solution obtained is then analyzed by UV-VIS spectrometry for the quantitative determination of the residual nicotinic acid, after a calibration curve obtained with appropriate standard solutions. The test is repeated in triplicate on the same sample.

Reconstituted artificial sweat absorption test: the test is conducted on a powdered material to determine the mass amount of nicotinic acid that this powder is able of capture. The execution of the test requires the use of three centrifuge tubes containing 2 g of powder product. For each tube, 15 ml of reconstituted artificial sweat is added. The suspensions are left under magnetic stirring overnight. The next day the tubes are centrifuged at 15000 rpm for 15 minutes. The remaining solid is weighted. The supernatant is transferred to a volumetric flask and suitably diluted. The solution obtained is then analyzed by UV-VIS spectrometry for the quantitative determination of the residual nicotinic acid, after a calibration straight line carried out with appropriate standard solutions. The test is repeated in triplicate on the same sample.

The UV-Vis spectroscopy technique can be used for determine the concentration of a substance dissolved in a solution. In order to use UV-Vis spectroscopy, it is necessary that the molecule contains a chromophore group, able of absorbing light in the ultraviolet or visible range. The Lambert-Beer law correlates the intensities of absorbed light with the substance concentrations. This machine, for example a double beam Jasco V-750, measures the absorbance of a molecule at a well-defined wavelength. This wavelength is usually selected considering the maximum of absorption in the molecule spectrum.

The X-ray diffraction technique is based on coherent elastic scattering: the macroscopic phenomenon of diffraction derives from the coherent sum of all the electromagnetic waves diffused by the atoms, that are located along the same family of lattice planes. To observe that phenomena, it is required the presence of a reticular order, as it is found in crystalline solids.

The technique used for the structural characterization of the solids described in that invention is the X-ray diffraction of polycrystalline powders, called XRPD spectroscopic analysis. The measurements were carried out with the Bruker D2 Phaser 2nd generation X-Ray Powder Diffractometer, with a theta-theta goniometer equipped with a LynxEye SSD160 solid state detector and using Cu Kα as a radiation source.

The samples were dry grounded using an agate mortar, until a homogeneous microfine powders were obtained. The samples were prepared with the lateral loading technique in order to eliminate the effects of preferential orientation of the crystals. The XRPD spectrum was recorded under the following conditions: - 2-theta (2θ) step size of 0.0101 °, time per step 0.5 s, ceramic tube with copper anode operating at 30 kV and 15 mA.

The scanning electron microscopy (SEM) technique uses as a radiation source an electron beam typically generated by a tungsten filament, which emits a flow of primary electrons focused by a series of electromagnetic lenses and deflected by an objective lens. The SEM, in addition to further refocusing the beam, imposes a controlled deflection on it, allowing the scanning of the sample areas. The effect produced by the interaction between electrons and matter is revealed and transformed into an electrical signal which, treated and amplified, is modulated into a television signal. These electrons are captured by a special detector and converted into electrical impulses sent in real time to a screen (a monitor) where a similar scan is performed simultaneously. The result is a black and white image with high resolution and large depth of field, which has characteristics similar to those of a normal photographic image. The magnification is given by the ratio between the size of the image and the size of the region that was scanned. Each solid analyzed was deposited on a sample holder and coated with a 10 nm thick layer of chromium through ionic sputtering. The SEM images reported in the invention were obtained using the Field emission scanning electron microscope (FE-SEM) LEO 1525 instrument equipped with an EDX Bruker probe.

EDX (Energy Dispersive X-ray Analysis) spectroscopy is an instrumental analytical method that uses the emission of X-rays generated by an accelerated electron beam incident on the sample. When the electron beam hits the sample, the electrons of the K or L layers are expelled and the system assumes a high-energy configuration. Following the electronic relaxation, an energy emission typical of each chemical element occurs. Therefore, the SEM analysis with the EDX probe allows for direct mapping of the chemical elements present in the analyzed sample and / or a semi-quantitative elemental analysis of the sample with the possibility of detecting the elements present in traces.

Using laser light diffraction technique (laser Diffraction Analysis) it is possible to measure the size and dimensional distribution of particles in powder, suspension or emulsion. This technology uses diffraction patterns (e.g. Mie theory) generated by a laser beam directed towards an object of dimensions comprises between nanometers and millimeters. The instrument used to carry out the measurements of the dimensions and the dimensional distribution of the solids object of the invention is the Malvern Mastersizer 2000 with Scirocco and Hydro G. This instrument provides dimensions and a diagram of the volume cumulative percentage of the particles, considered as "equivalent spherical diameter" (esd). The D₅₀, expressed in microns, corresponds to the average size of the particles and indicates that 50% of the analyzed particles have an equivalent spherical diameter lower than the value of D₅₀. D₁₀ and D₉₀ are determined with a similar method. Particle size can be determined using quality techniques described in ISO 13320-1 or any method equivalent to it.

The specific surface area (BET) of a solid indicates the surface area of the solid particles measured with respect to the unit mass and is expressed in m² / g. The surface area values reported in the invention were determined according to the BET method (Brunauer-Emmett-Teller model) through nitrogen physisorption measurements (measurement according to the BET method, AFNOR X 11-621 and 622 standard or ISO 9277).

Talc is a natural mineral used for a wide range of industrial purposes and in medical, pharmaceutical and cosmetic applications. In cosmetic field it is used as an absorber of greasy or aqueous components such as those of sweat, as a rheological modifier of formulations, as a whitening, mattifying or pearling agent. It is mainly used in nail polish, soaps, detergents, hair products, antiperspirants or deodorants and sun creams. Chemically, talc consists of a hydrated magnesium silicate Mg₃Si₄O₁₀ (OH) ₂ with a lamellar crystalline structure.

Historically and for many years, a particle size of the talc of around 44 microns has been considered of the optimum dimensions for the cosmetics applications. Recently, the size of the talc used in the cosmetic industry has been reduced thanks to new grinding technologies. Currently, the typical features of talc for cosmetic applications are: D₅₀ determined by laser diffraction generally between 10-20 µm, BET area between 3-22 m² / g, oil absorption ability between 20-100 ml / 100g.

The talc used as a reference in the present invention, has CAS number 14807-96-6, is microfine and really suitable for cosmetic applications, it has not been superficially treated, has a BET area of 4-6 m² / g, an oil absorption ability of 70-90 ml / 100 g, a tapped density of 0.5-0.6 g / cm³, a D₅₀ measured by laser diffraction between 10-25 µm. The X-ray diffraction spectrum obtained by XRPD powders has a first reflection at 2Θ = 9.4 ° ± 0.4 corresponding to an interlayer distance d = 9.4 ± 0.4 Å. The field of application of the present invention goes beyond the features of the talc used as a reference, and can be extended to other types of talc having different chemical-physical properties.

The first part of this invention describes a surface activation of the talc by chemical treatment. This activation can be obtained by surface erosion of the talc particles, treating them with aqueous solutions of acids or alkalis. In the context of the present invention, surface activation of talc particles means a surface morphological modification in order to increase the surface area of the particles by slightly modifying the particle size distribution. This morphological change is clearly visible by observing the SEM micrographs and measuring the BET area of the particles. This activation of particle surface has, as direct consequence, the increasing of the talc ability to absorb oil, in particular of the fat component of the sebum presents in body sweat.

The activation process by surface erosion can be obtained by treating talc with acidic aqueous solutions of hydrochloric acid, sulfuric acid, nitric acid or organic acids such as oxalic acid, citric acid, acetic acid, chloroacetic acid or lactic acid. The percentage concentration by mass of the acid in the solution is between 0.1-60%. The talc is dispersed in these solutions in a mass quantity comprises between 1% to 60%, or more preferably between 10% and 40%. The suspension of the talc in the acid solution is then heated to a temperature between 10-100 ° C, or more preferably between 40-80 ° C and kept under stirring for a period of between 1-72 h. At the end of the treatment, the acid suspension is neutralized with basic solutions containing hydroxides or oxides of metals and / or aliphatic or aromatic amines up to a pH between 5.0-7.5, then filtered and washed for several cycles until a washed paste product is obtained.

The activation of talc by surface erosion can also be obtained with basic aqueous solutions by dissolving hydroxides or metal oxides and / or aliphatic or aromatic amines. The mass concentration of the base in the solution is between 0.1-60%. The talc is dispersed in such solutions preferably in a mass quantity from 1% to 50%, or more preferably between 10% and 40%. The suspension of the talc in the alkaline solution is then heated to a temperature between 10-100 ° C, or more preferably between 40-80 ° C and kept under stirring for a period of between 1-72 h.

After the treatment, the acidic suspension is neutralized with basic solutions containing hydroxides or metal oxides and / or aliphatic or aromatic amines up to a pH between 5.0-7.5, then filtered and washed for several cycles until a washed paste product is obtained.

Activation of talc by surface etching can also be obtained with basic aqueous solutions by dissolving metal hydroxides or oxides and / or aliphatic or aromatic amines. The concentration by weight of the base in the solution is between 0.1-60%. The talc is dispersed in such solutions preferably in an amount between 1% to 50% by weight, or more preferably between 10% and 40%. The suspension of the talc in the alkaline solution is then heated to a temperature between 10°C and 100 ° C, or, preferably, between 40°C and 80 ° C and kept under stirring for 1-72 h. After the treatment, the basic suspension is neutralized with an acid such as hydrochloric acid, sulfuric acid, nitric acid or an organic acid such as acetic acid, citric acid or lactic acid up to a pH 5.0-7.5, then filtered and washed for several cycles until obtaining a washed paste product.

The effect of the activation of talc according to the present invention can be seen in Table 1.

**Table 1**

| **Sample** | **BET m2/g** | **D₅₀** | **Oil absorption capacity ml/100 g** | **Absorption capacity increase %** |
|---|---|---|---|---|
| Talc (reference sample) | 5,11 | 15,8 | 85,8 | 0 |
| Talc, activated with NaOH (10%) | 6,55 | 7,5 | 88,0 | + 2,5 |
| Talc, activated with HCl (10%) | 8,41 | 6,3 | 115,6 | + 34,8 |

Table 1 shows surface area values (BET), particle size distribution (D₅₀) and oil absorption capacity of the reference talc and of the talc treated with 10% by weight solutions of hydrochloric acid or sodium hydroxide for a period 24 hours at a temperature of 50 ° C. Surprisingly, it was found that, compared to the reference talc, activation by acidic treatment produces a considerable increase in the BET surface area which results in an increase in the ability of the talc to absorb oil (+ 34.8% compared to the reference), while the basic treatment produces a slight increase in surface area and oil absorption capacity (+ 2.5%).

The second part of the present invention deals with the deposition process of lamellar particles of zinc hydroxy chloride on the surface of the activated talc particles. After a neutralization and washing step, the zinc hydroxy chloride is added and mixed with a suspension of activated talc obtained as previously described. The mixing process is preferably carried out using a turbine with a rotation speed between 1500-3000 rpm, at temperatures between 10-60 ° C and for a time between 1-16 h, more preferably between 2-8 h.

Turbomixing is a necessary process to obtain an optimal dispersion of zinc hydroxy chloride in water. In particular, during this process, the shear forces produced by the turbine, combined with the hydration processes of the lamellar solid, allow the exfoliation of the zinc hydroxy chloride crystals in packets of lamellae having optimal dimensions for the deposition on the surface of the activated talc.

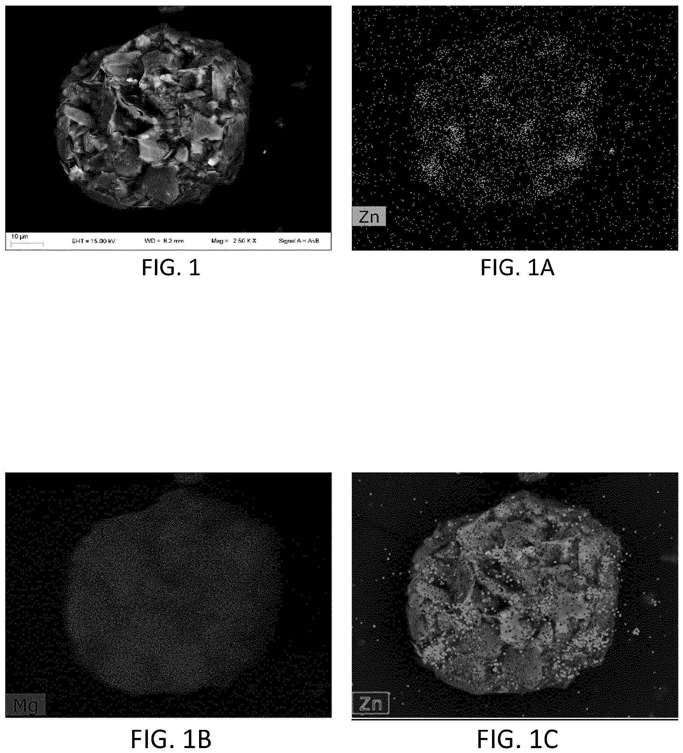

At the end of the turbomixing process, the mixture is dried by a spray-drying process. The evaporation process of the aqueous solvent through spray-drying contributes to the deposition of the zinc hydroxy chloride particles on the talc particles, which thanks to the ionic interactions are effectively deposited on the surface of the talc as can be seen in Fig. 1.

Fig. 1 shows an SEM image in which the regions with the highest zinc density are highlighted through the EDX technique (in green). The figure shows how the particles of zinc hydroxy chloride with an average D₅₀ of 3 µm are deposited on the surface of the larger particles of talc with an average D₅₀ of 10 µm.

Regarding the deposition process of zinc hydroxy chloride on the talc particles and the interactions between the materials, it should be noted that the lamellar solid zinc hydroxy chloride has a positive surface zeta potential, at pH between 5.0-7.0, in the range +60 - +20 mV as estimated from reference data (Junfeng He et al., Novel photocatalyst nitrogen-doped simonkolleite Zn5 (OH) 8Cl2 H2O with vis-up-conversion photoluminescence and effective visible-light, 2019, Appl. Phys. A), while the zeta potential of talc between pH 5.0 and 7.0 is estimated to be between -60 and -40 mV (B. Salopek, et al., Measurement and application of zeta-potential, 1992, Rudarsko-Geolosko-Naftni-Zbornik). The deposition process is therefore influenced by factors such as duration, temperature and speed of the turbine during the turbomixing process, the pH and therefore the Z potential of the two lamellar solids, the concentration of the solids in the suspension and their relative ratio, the drying process performed on the suspension.

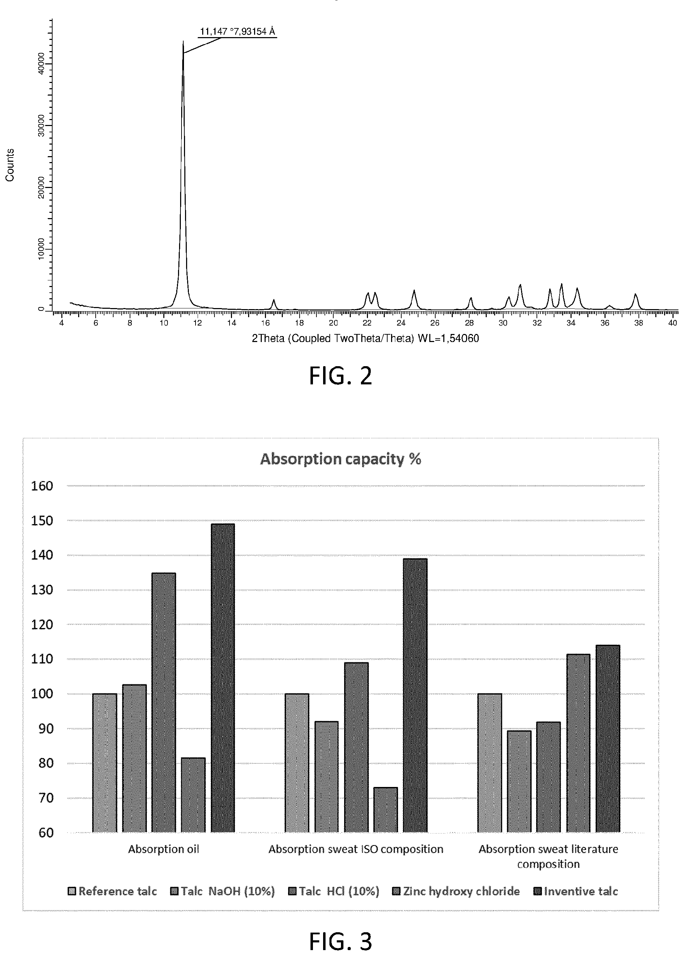

The zinc hydroxy chloride used in the present invention has a composition according to the formula Zn₅(OH)₈Cl₂ * xH₂O where x is between 0.5-4, a particle size distribution with D₉₀ <15 µm, D₅₀ <3.5 µm and D₁₀ <1,5 µm, a surface area between 1-30 m² / g, a monophasic crystal structure characterized by an XRPD crystallographic pattern in which a first reflection (d₀₀₃) is highlighted at 2Θ = 11.1 ° ± 0.4 with interlayer distance equal at 7.9 ± 0.4 Å, an anion exchange capacity of between 3-4 meq/g. Figure 2 shows the X-ray diffraction spectrum from powders of the zinc hydroxy chloride used in the present invention.

In the deposition process of the lamellar particles of zinc hydroxy chloride on the surface of the particles of the activated talc, the concentration by weight of zinc hydroxy chloride is between 1-50% with respect to the mass of the talc, or more preferably between 5-35% or still more preferably between 10-20%. On the other hand, the percentage by weight of the total inorganic solids with respect to the mass of the suspension is comprised between 5-60%, or more preferably between 10-40% or even more preferably between 20-30%.

After the activation process of the talc surface and the subsequent deposition process of the zinc hydroxy chloride particles, a new talc specialty was obtained with surprisingly increased absorption properties when compared with the reference talc. In particular, the talc obtained according to this invention shows an increase in the absorption capacity of oil, ISO-type sweat and reconstituted sweat.

Table 2 shows the absorption values of the reference talc, the reference talc after the activation process with 10% soda, the reference talc after the activation process with 10% hydrochloric acid and the reference talc after the activation with 10% hydrochloric acid, subsequent deposition process with 10% by weight of zinc hydroxy chloride particles on the surface of the talc and atomization.

**Table 2**

| **Sample** | **BET m²/g** | **D₅₀** | **Oil¹** | **Δ%²** | **Sweat ISO³** | **Δ %⁴** | **Sweat Rec.⁵** | **Δ%⁶** |
|---|---|---|---|---|---|---|---|---|
| Talc (reference sample) | 5,11 | 15,8 | 85,8 ± 0,1 | 0 | 130 ± 4 | 0 | 25 ± 2 | 0 |
| Talc, activated with NaOH (10%) | 6,55 | 7,5 | 88,0 ± 0,1 | + 2,5 | 122 ± 4 | -8.0 | 15 ± 5 | -10.7 |
| Talc, activated with HCl (10%) | 8,41 | 6,3 | 115,6 ± 0,1 | + 34,8 | 139 ± 4 | +9.0 | 17 ± 3 | -8.2 |
| Zinc hydroxy chloride | 20,1 | 3.4 | 70 ,0± 0,1 | - 18.4 | 103 + 5 | -27 | 37 ± 4 | +11.3 |
| TEA (Ex. N° 3)⁷ | 10.04 | 10.5 | 127,5 ± 0,1 | **+ 49** | 169 ± 5 | **+ 39.0** | 39 ± 2 | **+13.9** |

| | | | | | | | | |
|---|---|---|---|---|---|---|---|---|
| Uncertainty expressed as standard deviation. 1: Oil absorption capacity ml/100 g. 2: Δ % oil absorption capacity; 3: Absorption sweat following ISO procedure. 4: Δ % absorption sweat ISO. 5: absorption of reconstructed sweat. 6: Δ % absorption of reconstructed sweat. 7: Inventive talc obtained in example N° 3. | | | | | | | | |

The talc according to the invention of Example N° 3 consists of 90% by weight of talc activated through acid erosion and 10% of zinc hydroxy chloride particles deposited on the surface. Comparing the efficacy data in Table 2, it is to be noted that the absorption properties of talc according to the invention are not simply the algebraic sum calculated from the hypothetical physical mixture of talc activated by chemical erosion and zinc hydroxy chloride. In fact, the talc according to the invention shows peculiar and surprising absorption properties, due to the special production process that produces a synergistic effect between the activated talc and zinc hydroxy chloride. These results are summarized in the histogram graph of Fig. 3, where the absorption efficiency data reported in Table 2 have been normalized with respect to the reference talc and reported as a percentage change.

As can be seen from the graph in Fig. 3, the talc obtained according to the present invention has a remarkable higher absorbency than the reference talc. It should be emphasized that this significant increase is observed for all the components analysed: namely oil, ISO sweat and reconstituted artificial sweat.

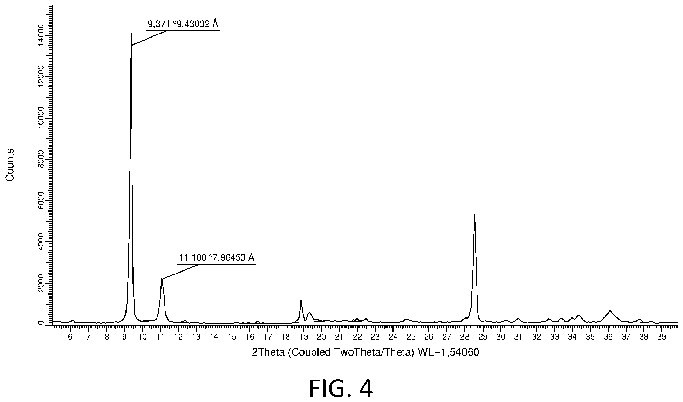

The distinctive chemical-physical characteristics of the surface-modified talc according to the invention (are the following: a D₅₀ between 1-30 µm, a BET surface area between 1-30 m²/g, an X-ray diffraction pattern having two clearly distinguishable signals in the XRPD spectrum: the first at 2Θ = 9.4 ° ± 0.4 and the second at 2Θ = 11.1 ° ± 0.4 (see Fig. 4), a morphology of the spheroidal particles in which they are distinguishable through SEM morphological analysis -EDX of the lamellar particles of zinc hydroxide chloride which partially coat the surface of the talc particles (see Fig. 1).

In addition to the increased absorbent properties, it was possible to verify that after the treatment according to the invention, the talc is faster and more easily dispersible in water than the reference talc, a considerable technical advantage from the point of view of industrial production of cosmetic end products.

All the characteristics of the surface modified talc, according to the above invention, make this product particularly suitable for use in cosmetic applications in the field of deodorants or antiperspirants to reduce body odour produced by the natural transpiration of the skin. The formulations in which it is possible to use the surface modified talc, according to the invention, are the same in which an untreated talc can be used, for example anhydrous formulations, aerosols, sticks, roll-ons, gels, emulgels, emulsions or creams.

The high efficiency of the surface modified talc according to the present invention opens up the possibility of creating effective deodorant cosmetic compositions without aluminium salts or with a reduced content of aluminium salts, giving the finished products greater safety for human health and better sensory properties.

### Example 1

Production process of surface activated talc through acid chemical erosion:
1) 100-150 g of talc are dispersed in 1 L of 10% hydrochloric acid solution.
2) The suspension is heated up to 60 ° C.
3) The suspension is left at 60 ° C under stirring for 10 hours.
4) The suspension is allowed to cool under stirring to room temperature.
5) Sodium hydroxide is added to the suspension until a pH between 5.0-7.5 is obtained.
6) The suspension is filtered and the talc washed twice with deionized water and subsequently dried.

### Example 2

Production process of surface activated talc through basic chemical erosion:
1) 100-150 g of talc are dispersed in 1 L of 10% sodium hydroxide solution.
2) The suspension is heated up to 60 ° C.
3) The suspension is left at 60 ° C under stirring for 10 hours.
4) The suspension is allowed to cool under stirring to room temperature.
5) Hydrochloric acid is added to the suspension until a pH between 5.0-7.5 is obtained.
6) The suspension is filtered and the talc washed twice with deionized water and subsequently dried.

### Example 3

Production process of talc with increased efficiency as described in the present invention with acid treatment:
1) 100-150 g of talc are dispersed in 1 L of 10% hydrochloric acid solution.
2) The suspension is heated up to 60 ° C.
3) The suspension is left at 60 ° C under stirring for 10 hours.
4) The suspension is allowed to cool under stirring to room temperature.
5) Sodium hydroxide is added to the suspension until a pH between 5.0-7.5 is obtained.
6) The suspension is filtered and the talc washed twice with deionized water (mass of water = 10x mass of talc), the washed talc is then re-suspended under turbo mixing in water to give a concentration of 20-30% by weight.
7) 10-15 g of zinc hydroxy chloride is added to the suspension.
8) It is turbomixed at 3000 rpm for 2-4 hours at 40 ° C.
9) The mixture is dried by spray-drying.

### Example 4

Formulation of a water-based roll-on deodorant without aluminium salts. This formulation can be obtained by simple mixing at room temperature and without the presence of oils or waxes.

| Raw material | % w/w |
|---|---|
| Water | 83,5 |
| Ethanol | 10 |
| Talc with increased efficiency | 5 |
| Phenoxyethanol | 0,9 |
| Ethylhexylglycerin | 0,1 |
| Carbomer | 0,2 |
| Sodium hydroxide 30% | 0,1 |
| Perfume | 0,2 |

### Example 5

Sprayable deodorant without gas and without aluminium salts. Formulation obtained under high shear force on cold solution.

| Raw material | % w/w |
|---|---|
| Water | 82,0 |
| Ethanol | 5,0 |
| Talc with increased efficiency | 0,9 |
| Phenoxyethanol | 0,1 |
| Cellulose gum, Microcrystalline cellulose | 0,5 |
| Triethylcitrate | 2,5 |
| Ethanol | 5,0 |
| Glycerine | 1,0 |
| PEG-40 Hydrogented castor oil | 2,0 |
| Perfume | 1,0 |

### Example 6

Alcohol spray deodorant without aluminium salts. Formulation obtained by simple mixing at room temperature.

| Raw material | % w/w |
|---|---|
| Denaturated ethanol (D type) | 94,4 |
| Fragrance | 1,5 |
| Triclosan | 0,1 |
| Talc with increased efficiency | 3,0 |
| Perfume | 1,0 |

### Example 7

Deodorant stick without aluminium salts. Formula obtained by melting the waxes at 75 ° C and subsequently mixing the powders. The suspension thus obtained is poured into the appropriate dispenser.

| Raw material | % w/w |
|---|---|
| Cyclopentasiloxane | 73,0 |
| Dimethicone | 2,0 |
| Cetyl alcohol | 15,0 |
| Polyethylene | 4,0 |
| Diisopropyl sebacate | 1,0 |
| Talc with increased efficiency | 5,0 |

### Example 8

Alcohol-free deodorant spray and without aluminium salts. The formulation is obtained by mixing the components cold and placing them in the appropriate pressure container.

| Raw material | % w/w |
|---|---|
| Cyclopentasiloxane | 10,0 |
| Dimethicone | 2,0 |
| Talc with increased efficiency | 5,0 |
| Propellant | 82 |
| Triethylcitrate | 5,0 |
| Perfume | 1,0 |

## Claims

1. A method of producing a surface modified talc comprising the following processes:
a. surface chemical erosion process of talc particles carried out by suspending the talc in an acidic aqueous solution;
b. deposition process of inorganic lamellar particles capable of anion exchange consisting of zinc hydroxy chloride on the surface of the talc particles obtained from process (a), carried out by adding said lamellar zinc hydroxy chloride particles to an aqueous suspension containing the talc obtained from process (a), subsequent mixing of the suspension containing the talc and the lamellar zinc hydroxy chloride particles and drying of the suspension.

2. Method according to claim 1, wherein the process (a) is carried out using an uncoated talc, with a particle size distribution determined by laser diffraction with a D₅₀ between 1-30 µm, with a BET surface area between 1-30 m²/g, an XRPD crystallographic pattern showing a first reflection (d₀₀₃) at 2Θ = 9.4 ° ± 0.4 corresponding to an interlayer distance of the lamellar solid of 9.4 ± 0.4 Å.

3. Method according to claims 1 or 2, wherein process (a) is carried out in acidic aqueous suspensions with percentages by mass of talc comprised between 1-60%, preferably between 10% and 40%, and percentages by mass of acid between 0.1-60%.

4. Method according to claim 3, wherein one or more of the following acids are used in process (a): hydrochloric acid, sulphuric acid, nitric acid, phosphoric acid, perchloric acid or organic acids such as acetic acid, citric acid or lactic acid, benzoic acid, benzene sulfonic acids, alkyl sulfonic acids, benzyl phosphonic or alkyl phosphonic acids.

5. Method according to any one of the preceding claims, wherein the process (a) is carried out at temperatures between 10-100 ° C, preferably between 40-80 °C and for a time between 1-72 hours.

6. Method according to any one of the preceding claims, wherein process (a), once the acid treatment step has been completed, also comprises a talc work-up step consisting of the following sub-steps:
i. neutralization step obtained by adjusting the pH to values between 5.0-7.5 using sodium hydroxide or metal hydroxide or alkylammonium hydroxide;
ii. washing step comprising one or more washing cycles in which a washing cycle consists of filtering the neutralized suspension using a filter press or solid-liquid separation system, obtaining a paste, suspending the paste in water and subsequent filtration as the previous one to obtain a washed paste;
iii. preparation step of a water suspension of the washed paste.

7. Method according to any one of the preceding claims, in which process (b) is carried out using lamellar zinc hydroxy chloride particles **characterized by** a composition according to the formula Zn₅(OH)₈Cl₂ x H₂O where x is between 0.5-4, a distribution of the particle sizes such that D₉₀ < 15 µm, D₅₀ < 3.5 µm e D₁₀ < 1,5 µm, a surface area BET between 1-30 m²/g, a monophasic crystal structure **characterized by** an XRPD crystallographic pattern in which shows a first reflection (d₀₀₃) at 2Θ = 11.1 ° ± 0.4 corresponding to an interlayer distance of the lamellar solid of 7.9 ± 0.4 Å and an anion exchange capacity between 3 and 4 meq / g.

8. Method according to any one of the preceding claims, wherein the process (b) is carried out by adding to the talc suspension a quantity by mass of zinc hydroxy chloride comprised between 1-50% with respect to the mass of talc, preferably between 5-35% or even more preferably between 10-20%.

9. Method according to claim 8, wherein said dispersion comprises a percentage by mass of inorganic particles, with respect to the total mass of the dispersion comprised between 5-60%, preferably between 10-40%, even more preferably between 20-30%.

10. Method according to any one of the preceding claims, in which process (b) is carried out using a turbomixer with turbine rotation speed between 1500-3000 rpm, at temperatures between 10-60 °C and with a duration between 1-16 hours or preferably between 2-8 hours.

11. Method according to any one of the preceding claims, wherein the superficially modified talc is obtained by drying the suspension through spray-drying.

12. Surface modified talc obtained with the method according to any one of the preceding claims, wherein said talc is **characterized by** a particle size with a D₅₀ between 1-30 µm, a spheroidal morphology of the particles, a BET surface area between values of 1-30 m²/g, having lamellar particles of zinc hydroxide chloride that partially cover the surface of the talc particles detectable through SEM-EDX morphological analysis and having clearly distinguishable diffraction signals in the XRPD spectrum at 2Θ = 9.4 ° ± 0.2 and at 2Θ = 11.2 ° ± 02.

13. Use of the superficially modified talc according to claim 12 for deodorant or antiperspirant cosmetic applications.

14. Use of the surface modified talc according to claim 12 for the production of an anhydrous cosmetic formulation containing at least one propellant.

15. Use of the superficially modified talc according to claim 12 for the realization of a cosmetic formulation stick, roll-on, gel, emulgel or cream.

## Patentansprüche

1. Verfahren zur Herstellung eines oberflächenmodifizierten Talks, umfassend die folgenden Prozesse:
a) chemischer Erosionsprozess an der Oberfläche von Talkteilchen, der durch Suspension des Talks in einer sauren, wässrigen Lösung erfolgt;
b) Prozess des Abscheidens anorganischer, lamellenförmiger Teilchen, die anionenaustauschfähig sind und aus Zinkhydroxychlorid auf der Oberfläche der Talkteilchen bestehen, die in einem Prozess (a) gewonnen werden, der durch Zugeben von lamellenförmigen Zinkhydroxychloridteilchen in eine wässrige Lösung erfolgt, die den im Prozess (a) gewonnenen Talk enthält, anschließendes Mischen der Suspension, die den Talk und die lamellenförmigen Zinkhydroxychloridteilchen enthält, und Trocknen der Suspension.

2. Verfahren nach Anspruch 1, wobei der Prozess (a) unter Verwendung eines nicht beschichteten Talks ausgeführt wird, mit durch Laserbeugung bestimmter Korngrößenverteilung mit einem D₅₀ zwischen 1-30 µm, einem BET-Oberflächenbereich zwischen 1-30 m²/g, einem kristallografischen Diffraktogramm, welches einen ersten Reflex (d₀₀₃) bei 2Θ = 9,4° ± 0,4 zeigt, der einem Zwischenschichtabstand des lamellenförmigen Feststoffs von 9,4 ± 0,4 Ä entspricht.

3. Verfahren nach den Ansprüchen 1 oder 2, wobei der Prozess (a) in sauren wässrigen Suspensionen erfolgt, mit Massenanteilen an Talk zwischen 1-60 %, vorzugsweise zwischen 10 % und 40 %, und Massenanteilen an Säure zwischen 0,1-60 %.

4. Verfahren nach Anspruch 3, wobei in dem Prozess (a) eine oder mehrere der folgenden Säuren verwendet werden: Salzsäure, Schwefelsäure, Salpetersäure, Phosphorsäure, Perchlorsäure oder organische Säuren wie Essigsäure, Zitronensäure, Milchsäure, Benzoesäure, Benzolsulfonsäuren, Alkylsulfonsäuren, Benzylphosphonsäuren oder Alkylphosphonsäuren.

5. Verfahren nach einem der vorstehenden Ansprüche, wobei der Prozess (a) bei Temperaturen zwischen 10-100 °C, vorzugsweise zwischen 40-80 °C über einen Zeitraum zwischen 1-72 Stunden erfolgt.

6. Verfahren nach einem der vorstehenden Ansprüche, wobei der Prozess (a) nach Abschluss des Säurebehandlungsschrittes auch einen Talk-Aufbereitungsschritt umfasst, der aus den folgenden Teilschritten besteht:
i. Neutralisierungsschritt, erhalten durch Einstellen des pH auf Werte zwischen 5,0-7,5 unter Verwendung von Alkylammoniumhydroxid;
ii. Waschschritt, umfassend einen oder mehrere Waschzyklen, wobei ein Waschzyklus aus der Filtration der neutralisierten Suspension unter Verwendung einer Filterpresse oder eines Feststoff-Flüssigkeits-Abscheidesystems, dem Erhalten einer Paste, erneutem Suspendieren der Paste in Wasser und anschließender Filtration wie der vorherigen besteht, um eine gewaschene Paste zu erhalten;
iii. Schritt der Zubereitung einer wässrigen Suspension der gewaschenen Paste.

7. Verfahren nach einem der vorstehenden Ansprüche, wobei der Prozess (b) unter Verwendung von lamellenförmigen Zinkhydroxychloridteilchen erfolgt, die **gekennzeichnet sind durch** eine Zusammensetzung nach der Formel Zn₅(OH)₈Cl₂·xH₂O, wobei x zwischen 0,5-4 liegt, die Teilchengrößenverteilung so ist, dass D₉₀ < 15 µm, D₅₀ < 3.5 µm und D₁₀ < 1,5 µm ist, ein BET-Oberflächenbereich zwischen 1-30 m²/g liegt, eine monophasische kristalline Struktur durch ein kristallografisches Diffraktogramm gekennzeichnet ist, welches einen ersten Reflex (d₀₀₃) bei 2Θ = 11,1° ± 0,4 zeigt, der einem Zwischenschichtabstand des lamellenförmigen Feststoffs von 7,9 ± 0,4 Ä und einer Anionenaustauschfähigkeit zwischen 3 und 4 meq/g entspricht.

8. Verfahren nach einem der vorstehenden Ansprüche, wobei der Prozess (b) erfolgt, indem der Talksuspension eine Massenmenge an Zinkhydroxychlorid zwischen 1-50 % relativ zur Talkmasse, vorzugsweise zwischen 5-35 % oder noch mehr bevorzugt zwischen 10-20 % zugegeben wird.

9. Verfahren nach Anspruch 8, wobei die Dispersion einen Massenanteil an anorganischen Teilchen relativ zur Gesamtmasse der Dispersion zwischen 5-60 %, vorzugsweise 10-40 %, noch mehr bevorzugt 20-30 % umfasst.

10. Verfahren nach einem der vorstehenden Ansprüche, wobei der Prozess (b) unter Verwendung eines Turbomischers mit einer Turbinendrehzahl zwischen 1500-3000 rpm bei einer Temperatur zwischen 10-60 °C und in einem Zeitraum zwischen 1-16 Stunden oder vorzugsweise zwischen 2-8 Stunden erfolgt.

11. Verfahren nach einem der vorstehenden Ansprüche, wobei der oberflächenmodifizierte Talk durch Trocknung der Suspension mittels Sprühtrockung gewonnen wird.

12. Oberflächenmodifizierter Talk, der mit dem Verfahren nach einem der vorstehenden Ansprüche gewonnen wird, wobei der Talk durch eine Teilchengröße mit einem D₅₀ zwischen 1-30 µm, einer kugelförmigen Morphologie der Teilchen, einem BET-Oberflächenbereich mit Werten zwischen 1-30 m²/g, mit lamellenförmigen Zinkhydroxychloridteilchen, die die Oberfläche der Talkteilchen teilweise bedecken, die durch morphologische SEM-EDX-Analyse nachweisbar sind und Beugungssignale aufweisen, die im XRPD-Spektrum bei 2Θ = 9,4°± 0,2 und bei 2Θ = 11,2° ± 02 klar erkennbar sind.

13. Verwendung des oberflächenmodifizierten Talks nach Anspruch 12 für kosmetische Deodorant- oder Antitranspirant-Anwendungen.

14. Verwendung des oberflächenmodifizierten Talks nach Anspruch 12 für die Herstellung einer wasserfreien kosmetischen Formulierung, die mindestens ein Treibmittel enthält.

15. Verwendung des oberflächenmodifizierten Talks nach Anspruch 12 für die Herstellung einer kosmetischen Stick-, Roller-, Gel-, Emulgel- oder Creme-Formulierung.

## Revendications

1. Méthode de production d'un talc modifié en surface comprenant les phases suivantes :
a) processus d'érosion chimique de la surface de particules de talc effectué en mettant en suspension le talc dans une solution aqueuse acide ;
b) processus de déposition de particules inorganiques lamellaires capables d'échange anionique constituées de chlorure de zinc hydroxy sur la surface des particules de talc obtenues moyennant le processus (a), réalisé en ajoutant lesdites particules lamellaires de chlorure de zinc hydroxy à une suspension aqueuse contenant le talc obtenu moyennant le processus (a), ultérieur mélange de la suspension contenant le talc et des particules lamellaires de chlorure de zinc hydroxy et séchage de la suspension.

2. Méthode selon la revendication 1, où le processus (a) est réalisé en utilisant un talc non revêtu, avec distribution granulométrique déterminée par diffraction laser avec un D₅₀ compris entre 1-30 µm, avec une zone de surface BET comprise entre 1-30 m²/g, un modèle cristallographique XRPD montrant une première réflexion (d₀₀₃) à 2Θ = 9,4° ± 0,4 correspondante à une distance d'intercouche du solide lamellaire de 9,4 ± 0,4 Å.

3. Méthode selon les revendications 1 ou 2, où le processus (a) est réalisé dans des suspensions aqueuses acides exprimées en pourcentages en masse de talc compris entre 1-60%, préférablement entre 10% et 40%, et pourcentages en masse d'acide compris entre 0,1-60%.

4. Méthode selon la revendication 3, où dans le processus (a) on utilise l'un ou plusieurs des acides suivants : acide chloridrique, acide sulfurique, acide nitrique, acide phosphorique, acide perchlorique ou des acides organiques comme l'acide acétique, l'acide citrique ou l'acide lactique, l'acide benzoïque, les acides benzènes sulfoniques, les acides alkyls sulfoniques, les acides benzyles phosphoniques ou alkyls phosphoniques.

5. Méthode selon l'une quelconque des revendications précédentes, où le processus (a) est réalisé à des températures comprises entre 10-100°C, préférablement entre 40-80°C et pour une durée comprise entre 1-72 heures.

6. Méthode selon l'une quelconque des revendications précédentes, où le processus (a), lorsque la phase de traitement acide est conclue, comprend également une phase de préparation constituée des sous-phases suivantes :
i. phase de neutralisation obtenue en ajustant le pH à des valeurs comprises entre 5,0-7,5 en utilisant de l'hydroxyde de sodium ou de l'hydroxyde de métal ou de l'hydroxyde d'alkylammonium ;
ii. phase de lavage comprenant un ou plusieurs cycles de lavage où un cycle de lavage consiste dans la filtration de la suspension neutralisée en utilisant un filtre-presse ou un système de séparation solide-liquide, en obtenant une pâte, nouvelle suspension de la pâte dans de l'eau et successive filtration comme la précédente pour obtenir une pâte lavée ;
iii. phase de préparation d'une suspension de la pâte lavée dans de l'eau.

7. Méthode selon l'une quelconque des revendications précédentes, où le processus (b) est réalisé en utilisant des particules lamellaires de chlorure de zinc hydroxy **caractérisées par** une composition selon la formule Zn₅(OH)₈Cl₂·xH₂O où x est compris entre 0,5-4, une distribution des dimensions particulaires telles que D₉₀ < 15 µm, D₅₀ < 3,5 µm et D₁₀ < 1,5 µm, une zone de surface BET comprise entre 1-30 m²/g, une structure cristalline monophasique **caractérisée par** un modèle cristallographique XRPD montrant une première réflexion (d₀₀₃) à 2Θ = 11,1° ± 0,4 correspondante à une distance d'intercouche du solide lamellaire de 7,9 ± 0,4 Å et une capacité d'échange anionique comprise entre 3 et 4 m²/g.

8. Méthode selon l'une quelconque des revendications précédentes, où le processus (b) est réalisé en ajoutant à la suspension de talc une quantité en masse de chlorure de zinc hydroxy comprise entre 1-50% par rapport à la masse de talc, préférablement entre 5-35% ou encore plus préférablement entre 10-20%.

9. Méthode selon la revendication 8, où ladite dispersion comprend un pourcentage en masse de particules inorganiques, par rapport à la masse totale de la dispersion, compris entre 5-60%, préférablement entre 10-40%, encore plus préférablement entre 20-30%.

10. Méthode selon l'une quelconque des revendications précédentes, où le processus (b) est réalisé moyennant un agitateur turbinaire ayant vitesse de rotation de la turbine comprise entre 1500-3000 tr/min, à des températures comprises entre 10-60°C et pour des durées comprises entre 1-16 heures ou préférablement entre 2-8 heures.

11. Méthode selon l'une quelconque des revendications précédentes, où le talc modifié en surface est obtenu par séchage de la suspension moyennant séchage par atomisation.

12. Talc modifié en surface obtenu avec la méthode selon l'une quelconque des revendications précédentes, où ledit talc est **caractérisé par** une dimension des particules avec un D₅₀ compris entre 1-30 µm, une morphologie sphéroïdale des particules, une zone de surface BET comprise entre les valeurs de 1-30 m²/g, ayant des particules lamellaires de chlorure de zinc hydroxy qui revêtent partiellement la surface des particules de talc relevables à travers une analyse morphologique SEM-EDX et ayant des signaux de diffraction clairement distinguables dans le spectre XRPD à 2Θ = 9,4°± 0,2 et à 2Θ = 11,2° ± 02.

13. Usage du talc modifié en surface selon la revendication 12 pour des applications cosmétiques déodorantes ou antiperspirantes.

14. Usage du talc modifié en surface selon la revendication 12 pour la réalisation d'une formulation cosmétique anhydre contenant au moins un produit propulseur.

15. Usage du talc modifié en surface selon la revendication 12 pour la réalisation d'une formulation cosmétique en stick, à bille, gel, émulgel ou crème.
